# EUROPÄISCHE PATENTSCHRIFT

(11) **EP 3 188 801 B1**
(45) Veröffentlichungstag und Bekanntmachung des Hinweises auf die Patenterteilung: **01.05.2019**
(21) Anmeldenummer: 15730807.3
(22) Anmeldetag: 24.06.2015
(51) Int. Cl.: A61Q 5/06, A61K 8/81

(54) **MITTEL UND VERFAHREN ZUR TEMPORÄREN VERFORMUNG KERATINHALTIGER FASERN**
PRODUCTS AND METHOD FOR THE TEMPORARY SHAPING OF KERATIN-CONTAINING FIBERS
PRODUITS ET PROCÉDÉ DE MISE EN FORME TEMPORAIRE DE FIBRES KÉRATINIQUES

(30) Priorität: 03.09.2014 DE 102014217602
(43) Veröffentlichungstag der Anmeldung: 12.07.2017
(73) Patentinhaber: Henkel AG & Co. KGaA, 40589 Düsseldorf (DE)
(72) Erfinder: LANGE, Julia, Bibiane, 24641 Sievershütten (DE); RICHTERS, Bernd, 21129 Hamburg (DE); BERMUDEZ AGUDELO, Maria, Catalina, 64295 Darmstadt (DE); MARTINEZ, Cyrielle, 22765 Hamburg (DE)
(86) Internationale Anmeldenummer: PCT/EP2015/064184
(87) Internationale Veröffentlichungsnummer: WO 2016/034300

(56) Entgegenhaltungen:
- WO-A1-2011/017223
- Laurie J. Marshall, et al.: "Unique Styling Products Via Maltodextrin/VP", happi , 6. November 2012 (2012-11-06), Seite 10PP, XP002743292, Gefunden im Internet: URL:http://www.happi.com/contents/view_fea tures/2012-11-06/unique-styling-products-v ia-maltodextrinvp/ [gefunden am 2015-08-12]
- Anonmous: "STRUCTURE PLUS", National Starch & Chemical , 2001, XP002743293, Gefunden im Internet: URL:http://www.researchgate.net/publictopi cs.PublicPostFileLoader.html?id=50576ae9e2 4a463e21000036&key=9fcfd50576ae9757af [gefunden am 2015-08-12]

## Beschreibung

Die vorliegende Erfindung betrifft ein kosmetisches Mittel zur Haarfestigung bzw. zur temporären Umformung von keratinischen Fasern, insbesondere menschlichen Haaren, wobei das Mittel eine Kombination von zwei Polymeren enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, Festigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen.

Ein beispielhaftes Stylingmittel mit guten Halteeigenschaften und hoher Feuchtebeständigkeit auf Grundlage einer Kombination aus einem hydrophob modifizierten (Meth)acrylsäure Copolymer und einem hydrophob modifizierten Polysaccharid wird in der internationalen Patentanmeldung WO2014/26804A2 beschrieben.

Zur temporären Haarverformung geeignete Copolymere, die durch Umsetzung ethylenisch ungesättigter Monomereinheiten mit saccharidischen Monomereinheiten, beispielsweise durch Umsetzung von Vinylpyrrolidon mit Maltodextrin erhalten werden, werden in der internationalen Anmeldung WO 2011/017223 A1 beschrieben.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie vorzugsweise stabil transparenter kosmetischer Zusammensetzungen eignen. Insbesondere sind derzeit erhältliche Stylingmittel noch dahingehend verbesserbar, dass eine gute Kombination aus Steifheit (Stiffness) und Langzeithalt nicht immer ausreichend gewährleistet wird. Es ist daher eine Aufgabe der vorliegenden Erfindung, derartige Stylingmittel bereitzustellen, die neben den oben genannten Eigenschaften insbesondere sowohl eine gute Steifheit als auch einen guten Langzeithalt ergeben.

Diese Aufgabe konnte durch die Kombination zweier spezifischer Polymere gelöst werden. Ein erster Gegenstand der vorliegenden Anmeldung ist daher ein kosmetisches Mittel zur temporären Verformung keratinischer Fasern, enthaltend, bezogen auf sein Gesamtgewicht
a) 0,5 bis 3,0 Gew.-% mindestens eines hydrophob modifizierten (Meth)acrylsäure Copolymers, welches durch Umsetzung
   - mindestens eines Monomers (a1) aus der Gruppe Acrylsäure, Methacrylsäure, C₁-C₆-Alkylacrylsäureester, C1-C6-Alkylmethacrylsäureester, mit
   - mindestens einem Monomer (a2) aus der Gruppe der C₁₀₋₃₀ Alkyl Acrylate, C10-30 Alkyl Methacrylate erhalten wird
b) 0,5 bis 5,0 Gew.-% mindestens eines, von dem Copolymer a) verschiedenen Copolymers, welches durch Umsetzung
   - mindestens eines Monomers (b1) aus der Gruppe N-Vinylpyrrolidon und N-Vinylformamid, mit
   - mindestens einem Monomer (b2) aus der Gruppe der modifizierten Stärken, vorzugsweise der hydrolysierten Stärken, besonders bevorzugt aus der Gruppe der Maltodextrine erhalten wird.

Ein erster wesentlicher Bestandteil erfindungsgemäßer kosmetischer Mittel ist das hydrophob modifizierte (Meth)acrylsäure Copolymer a). Bevorzugte Copolymere a) haben eine verdickende Wirkung.

Als hydrophob modifizierte (Meth)acrylsäure Copolymere a) werden vorzugsweise Copolymere verwendet, die sich auf
- mindestens ein Monomer (a1) aus der Gruppe der ungesättigten Carbonsäuren und ungesättigten Carbonsäureester, und
- mindestens ein Monomer (a2) aus der Gruppe der ungesättigten hydrophob modifizierten Monomere.
zurückführen lassen.

Copolymere a) basieren auf mindestens einem Monomer (a1) aus der Gruppe Acrylsäure, Methacrylsäure, C₁-C₆-Alkylacrylsäureester, C₁-C₆-Alkylmethacrylsäureester. Bei den Acrylsäureestern und Methacrylsäureestern handelt es sich um Ester der jeweiligen Säuren mit nichttertiären Alkylalkoholen mit Alkylresten von 1 bis 6 Kohlenstoffatomen, insbesondere 2 bis 4 Kohlenstoffatomen. Als geeignete Monomere seien beispielsweise Ethylacrylat, Ethylmethacrylat, Propylacrylat, Propylmethacrylat, n-Butylacrylat, n-Butylmethacrylat, Isobutylacrylat und 2-Methylbutylacrylat genannt.

Die Gruppe der hydrophob modifizierten Monomere (a2) bezeichnet Monomere, die über eine hydrophobe Teilstruktur verfügen. Monomere (a2) lassen sich ihrerseits auf die zwei nachfolgenden Struktureinheiten zurückführen:
- eine Acrylsäure oder Methacrylsäure
- eine C₁₀₋₃₀Alkylkette,

Als Monomer (a2) werden beispielsweise C₁₀₋₃₀Alkyl Acrylate, eingesetzt. Zusammenfassend werden Copolymere a) bevorzugt, die aus
- mindestens einem Monomer (a1) aus der Gruppe Acrylsäure, Methacrylsäure, C₁-C₆-Alkylacrylsäureester, C₁-C₆-Alkylmethacrylsäureester,
- mindestens einem Monomer (a2) aus der Gruppe der der C₁₀₋₃₀Alkyl Acrylate, gebildet werden.

Besonders bevorzugt werden Copolymere a), die durch Umsetzung
- mindestens eines Monomers (a1) aus der Gruppe Acrylsäure, Methacrylsäure, C₁-C₆-Alkylacrylsäureester, C₁-C₆-Alkylmethacrylsäureester, mit
- mindestens einem Monomer (a2) aus der Gruppe der C₁₀₋₃₀Alkyl Acrylate, C₁₀₋₃₀Alkyl Methacrylate, erhalten werden.

Weitere bevorzugte hydrophob modifizierte (Meth)acrylsäure Copolymere a) werden außer aus den zuvor beschreibenen Monomeren (a1) und (a2) aus mindestens einem Monomer (a3) aus der Gruppe der ungesättigten Amingruppen-haltigen Monomere gebildet.

Als Monomer (a3) werden vorzugsweise Monomere aus der Gruppe Acrylamid, Methacrylamid, Mono-(C₁-C₄)-alkylamino(C₁-C₄)-alkylacrylat, Di-(C₁-C₄)-alkylamino(C₁-C₄)-alkylacrylat, Mono-(C₁-C₄)-alkylamino(C₁-C₄)-alkylmethacrylat, Di-(C₁-C₄)-alkylamino(C₁-C₄)-alkylmethacrylat eingesetzt. Beispielhafte und bevorzugte Monomer (A2) sind 2-(N,N-Dimethylamino)ethyl Acrylat, 2-(N,N-Dimethylamino)ethyl Methacrylate, 2-(N,N-Diethylamino)ethyl Acrylate, 2-(N,N-Diethylamino)ethyl Methacrylate, 3-(N,N-Dimethylamino)propyl-Acrylat, 3-(N,N-Dimethylamino)propyl-Methacrylat, 2-(N,N-Dimethylamino)neopentyl Acrylat, N'-(3-N,N-Dimethylamino)propyl-Acrylamid, N'-(3-N,N-Dimethylamino)propyl-Methacrylamid.

Bevorzugt eingesetzte Copolymere a) sind nicht quervernetzt.

Zusammenfassend sind erfindungsgemäß bevorzugte kosmetische Mittel dadurch gekennzeichnet, dass das Copolymer a) ausgewählt ist aus der Gruppe der Verbindungen mit den INCI Bezeichnungen Acrylates/C₁₀₋₃₀-Alkyl Methacrylate Copolymer, Acrylates/C₁₀₋₃₀-Alkyl Acrylate Crosspolymer, Entsprechende Polymere sind beispielsweise unter den Handelsnamen Luvigel® FIT, Ultrez® 21, Pemulen® TR1 ,Aculyn® 22, Aculyn® 28, Aculyn® 88, Structure® 2001, Structure® 3001, Synthalen® W2000 und Structure® Plus erhältlich. Mit besonderem Vorzug ist das Copolymer a) ausgewählt aus der Gruppe der Verbindungen mit der INCI Bezeichnung Acrylates/C₁₀₋₃₀-Alkyl Methacrylate Copolymer.

Als für die kosmetische Wirkung erfindungsgemäßer Mittel hat es sich als vorteilhaft erwiesen, den Gewichtsanteil des Copolymers a) am Gesamtgewicht des kosmetischen Mittels weiter zu begrenzen. Bevorzugte kosmetische Mittel sind daher dadurch gekennzeichnet, dass der Gewichtsanteil des Copolymers a) am Gesamtgewicht des kosmetischen Mittels 0,7 bis 2,0 Gew.-%, vorzugsweise 0,9 bis 1,2 Gew.-% beträgt.

Als zweiten wesentlichen Bestandteil enthalten die erfindungsgemäßen kosmetischen Mittel mindestens ein, von dem Copolymer a) verschiedenen Copolymer b), welches durch Umsetzung mindestens einer ethylenisch ungesättigten Monomereinheit (b1) mit mindestens einer saccharidischen Monomereinheit (b2) erhalten wird. Bevorzugte Copolymere b) wirken nicht verdickend. Der Gewichtsanteil des Copolymers b) am Gesamtgewicht des kosmetischen Mittels beträgt vorzugsweise 1,0 bis 4,0 Gew.-%, besonders bevorzugt 1,5 bis 3,0 Gew.-%.

Die Copolymere b) werden vorzugsweise durch Umsetzung einer wachsenden synthetischen Polymerkette der ungesättigten Monomereinheit (b1) mit einer saccharidischen Monomereinheit (b2) hergestellt. Es wird angenommen, dass die Reaktion über einen Mechanismus erfolgt, bei welchem
- mittels eines Initiators freie Radikale gebildet werden, die mit der ungesättigten Monomereinheit (b1) reagieren und die Bildung einer synthetischen Polymerkette aus Monomereinheiten (b1) initiiert
- die Kettenübertragung unter Ausbildung eines (b1)-Homopolymers u.a. durch Abstraktion eines Protons aus der saccharidischen Monomereinheit (b2) beendet wird
- das resultierende Radikal der saccharidischen Monomereinheit (b2) mit einer ungesättigten Monomereinheit (b1) reagiert und auf diese Weise die Bildung des Copolymers b) initiiiert.

Ein abreagiertes Reaktionsgemisch der Monomereinheiten (b1) und (b2) wird in der Regel neben dem Copolymer b) weiterhin u.a. (b1)-Homopolymer sowie Reste des saccharidischen Monomers b2) enthalten.

Die saccharidische Monomereinheit (b2) wird in Folge des zuvor beschriebenen Reaktionsmechanismus in der Regel die Endgruppe des Copolymers b) bilden.

Das Copolymer b) ist vorzugsweise löslich in Wasser und Wasser/Ethanol-Gemischen. Die Löslichkeit in Wasser (25°C) beträgt vorzugsweise mehr als 1g/l, bevorzugt mehr als 10g/l.

Als ethylenisch ungesättigte Monomereinheiten (b1) nichtionische Monomere eingesetzt. Zur Gruppe der nichtionischen Monomere (b1) zählen N-Vinylpyrrolidon und N-Vinylformamid.

Das nicht-ionische ethylenisch ungesättigte Monomer (b2) ist vorzugsweise wasserlöslich.

Als saccharidische Monomereinheiten (b2) können Monosaccharide, Disaccharide aber auch Oligo- und Polysaccharide einsetzt werden. Geeignete Polysaccharide sind Stärken.

Besonders bevorzugt ist der Einsatz von Stärke und Stärkederivate, beispielsweise der Einsatz des durch Hydrolyse von Stärke erhaltene Maltodextrins oder des durch Erhitzen von Stärke erhältlichen Pyrodextrins.

Bei dem durch Hydrolyse von Stärke erhältlichen Maltodextrin handelt es sich um ein wasserlösliches Kohlenhydratgemisch aus Monomeren, Dimeren, Oligomeren und Polymeren der Glucose. Der Einsatz von Maltodextrin als Monomer (b2) ist besonders bevorzugt.

Copolymere b) werden zusammenfassend damit durch Umsetzung
- mindestens eines Monomers (b1) aus der Gruppe N-Vinylpyrrolidon und N-Vinylformamid, vorzugsweise N-Vinylpyrrolidon, mit
- mindestens einem Monomer (b2) aus der Gruppe der modifizierten Stärken, vorzugsweise der hydrolysierten Stärken, besonders bevorzugt aus der Gruppe der Maltodextrine
erhalten wird.

Eine besonders bevorzugte Ausführungsform erfindungsgemäßer kosmetischer Mittel ist dadurch gekennzeichnet, dass das Copolymer b) aus der Gruppe der Verbindungen mit der INCI Bezeichnung Maltodextrin/VP Copolymer ausgewählt ist.

Neben den zuvor beschriebenen Copolymeren a) und Copolymeren b) können die erfindungsgemäßen kosmetischen Mittel weitere Wirk-, Hilfs- und Pflegestoffe enthalten.

Eine erste Gruppe mit Vorzug eingesetzter Wirkstoffe sind die filmbildenden Polymere. Diese filmbildenden Polymere sind dabei nicht identisch mit dem zuvor beschriebenen hydrophob modifizierten (Meth)acrylsäure Copolymer a). Der Gewichtsanteil des filmbildenden Polymers am Gesamtgewicht der kosmetischen Mittel beträgt vorzugsweise 0,1 bis 8,0 Gew.-%, bevorzugt 0,5 bis 6,0 Gew.-% und inbesondere 1,0 bis 4,0 Gew.-%.

Als filmbildende Polymere werden mit besonderem Vorzug nichtionische Polymere eingesetzt. Geeignete nichtionische Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack.
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Aufgrund ihrer kosmetischen Wirkung in Kombination mit den Copolymeren a) und b) erfindungsgemäß bevorzugt eingesetzte filmbildenden Polymere sind insbesondere die Polyvinylpyrrolidone (INCI-Bezeichnung: PVP) sowie die Vinylpyrrolidon/Vinylacetat-Copolymere (INCI-Bezeichnung VP/VA Copolymer), wobei der Gewichtsanteil dieser Polymere vorzugsweise auf Mengen zwischen 1,0 und 10 Gew.-% beschränkt wird. Besonders bevorzugte erfindungsgemäße kosmetische sind daher dadurch gekennzeichnet, dass sie bezogen auf ihr Gesamtgewicht weiterhin 1,0 bis 10 Gew.-% Polyvinylpyrrolidon und/oder Vinylpyrrolidon/Vinylacetat-Copolymer, vorzugsweise Polyvinylpyrrolidon enthalten. Besonders bevorzugter kosmetische Mittel weisen einen Gewichtsanteil des Polyvinylpyrrolidons und/oder Vinylpyrrolidon/Vinylacetat-Copolymers c) am Gesamtgewicht des kosmetischen Mittels von 2,0 bis 8,5 Gew.-%, vorzugsweise von 3,0 bis 7,0 Gew.-% auf.

Zusammenfassend enthalten erfindungsgemäß besonders bevorzugte kosmetische Mittel mit den Copolymeren a) und b) sowie dem filmbildenden Polymer c) drei voneinander verschiedene Polymere. In einer bevorzugten Ausführungsform enthalten die kosmetischen Mittel bezogen auf ihr Gesamtgewicht
a) 0,5 bis 3,0 Gew.-% eines Copolymers a), welches durch Umsetzung
   - mindestens eines Monomers (A1) aus der Gruppe Acrylsäure, Methacrylsäure, C₁-C₆-Alkylacrylsäureester, C1-C6-Alkylmethacrylsäureester, mit
   - mindestens einem Monomer (A2) aus der Gruppe der der C₁₀₋₃₀Alkyl Acrylate, C₁₀₋₃₀Alkyl Methacrylate, erhaltend wird;
b) 0,5 bis 5,0 Gew.-% eines Copoymers b), welche durch Umsetzung von N-Vinylpyrrolidon (B1) mit mindestens einem Monomer (B2) aus der Gruppe der Maltodextrine erhalten wird;
c) 1,0 bis 10 Gew.-% Polyvinylpyrrolidon.

Besonders bevorzugt sind kosmetische Mittel, die, bezogen auf ihr Gesamtgewicht
a) 0,9 bis 1,2 Gew.-% eines Copolymer a) aus der Gruppe der Verbindungen mit der INCI Bezeichnung Acrylates/C10-30 Alkyl Methacrylate Copolymer;
b) 1,5 bis 3,0 Gew.-% eines Copolymers b) aus der Gruppe der Verbindungen mit der INCI Bezeichnung Maltodextrin/VP Copolymer;
c) 3,0 bis 7,0 Gew.-% Polyvinylpyrrolidon
enthalten.

Als Pflegestoff können Proteinhydrolysate und/oder deren Derivate eingesetzt werden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Ein weitere Gruppe von Pflegestoffen sind die Vitamine, Provitamine, Vitaminvorstufen und/oder deren Derivate. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Glycerin, Propylenglykol, Panthenol, Coffein, Nicotinamid und Sorbitol.

Als Pflegestoff können Pflanzenextrakt, aber auch Mono- bzw. Oligosaccharide und/oder Lipide eingesetzt werden.

Bevorzugte kosmetische Mittel basieren auf einem wässrigen oder wässrig/alkoholischen Träger. Der Wasseranteil am Gesamtgewicht erfindungsgemäßer kosmetischer Mittel beträgt vorzugsweise 80 bis 99 Gew.-%, bevorzugt 84 bis 98 Gew.-% und insbesondere 90 bis 97 Gew.-%.

Die Zusammensetzung einiger verwendeter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Optionale Additive | add 100 | add 100 | add 100 | add 100 | add 100 |

| | Formel 6 | Formel 7 | Formel 8 | Formel 9 | Formel 10 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 11 | Formel 12 | Formel 13 | Formel 14 | Formel 15 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Optionale Additive | add 100 | add 100 | add 100 | add 100 | add 100 |

| | Formel 16 | Formel 17 | Formel 18 | Formel 19 | Formel 20 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 21 | Formel 22 | Formel 23 | Formel 24 | Formel 25 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Optionale Additive | add 100 | add 100 | add 100 | add 100 | add 100 |

| | Formel 26 | Formel 27 | Formel 28 | Formel 29 | Formel 30 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 31 | Formel 32 | Formel 33 | Formel 34 | Formel 35 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Optionale Additive | add 100 | add 100 | add 100 | add 100 | add 100 |

| | Formel 36 | Formel 37 | Formel 38 | Formel 39 | Formel 40 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 41 | Formel 42 | Formel 43 | Formel 44 | Formel 45 |
|---|---|---|---|---|---|
| Acrylates/C₁₀₋₃₀ Alkyl Methacrylate Copolymer | 0,5 bis 3,0 | 0,5 bis 3,0 | 0,7 bis 2,0 | 0,7 bis 2,0 | 0,9 bis 1,2 |
| Maltodextrin/VP Copolymer | 0,5 bis 5,0 | 1,0 bis 4,0 | 1,0 bis 4,0 | 1,5 bis 3,0 | 1,5 bis 3,0 |
| Optionale Additive | add 100 | add 100 | add 100 | add 100 | add 100 |

| | Formel 46 | Formel 47 | Formel 48 | Formel 49 | Formel 50 |
|---|---|---|---|---|---|
| Acrylates/C₁₀₋₃₀ Alkyl Methacrylate Copolymer | 0,5 bis 3,0 | 0,5 bis 3,0 | 0,7 bis 2,0 | 0,7 bis 2,0 | 0,9 bis 1,2 |
| Maltodextrin/VP Copolymer | 0,5 bis 5,0 | 1,0 bis 4,0 | 1,0 bis 4,0 | 1,5 bis 3,0 | 1,5 bis 3,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 51 | Formel 52 | Formel 53 | Formel 54 | Formel 55 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Optionale Additive | add 100 | add 100 | add 100 | add 100 | add 100 |

| | Formel 56 | Formel 57 | Formel 58 | Formel 59 | Formel 60 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 61 | Formel 62 | Formel 63 | Formel 64 | Formel 65 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Optionale Additive | add 100 | add 100 | add 100 | add 100 | add 100 |

| | Formel 66 | Formel 67 | Formel 68 | Formel 69 | Formel 70 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ²⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 71 | Formel 72 | Formel 73 | Formel 74 | Formel 75 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Optionale Additive | add 100 | add 100 | add 100 | add 100 | add 100 |

| | Formel 76 | Formel 77 | Formel 78 | Formel 79 | Formel 80 |
|---|---|---|---|---|---|
| Copolymer a) ¹⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 81 | Formel 82 | Formel 83 | Formel 84 | Formel 85 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Optionale Additive | add 100 | add 100 | add 100 | add 100 | add 100 |

| | Formel 86 | Formel 87 | Formel 88 | Formel 89 | Formel 90 |
|---|---|---|---|---|---|
| Copolymer a) ³⁾ | 0,5 bis 3,0 | 0,5 bis 3,0 | 0,7 bis 2,0 | 0,7 bis 2,0 | 0,9 bis 1,2 |
| Copolymer b) ⁴⁾ | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | Formel 91 | Formel 92 | Formel 93 | Formel 94 | Formel 95 |
|---|---|---|---|---|---|
| Acrylates/C₁₀₋₃₀ Alkyl Methacrylate Copolymer | 0,5 bis 3,0 | 0,5 bis 3,0 | 0,7 bis 2,0 | 0,7 bis 2,0 | 0,9 bis 1,2 |
| Maltodextrin/VP Copolymer | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Optionale Additive | add 100 | add 100 | add 100 | add 100 | add 100 |

| | Formel 96 | Formel 97 | Formel 98 | Formel 99 | Formel 100 |
|---|---|---|---|---|---|
| Acrylates/C₁₀₋₃₀ Alkyl Methacrylate Copolymer | 0,5 bis 3,0 | 0,5 bis 3,0 | 0,7 bis 2,0 | 0,7 bis 2,0 | 0,9 bis 1,2 |
| Maltodextrin/VP Copolymer | 0,5 bis 5,0 | 1,0 bis 4,0 | 1,0 bis 4,0 | 1,5 bis 3,0 | 1,5 bis 3,0 |
| Polyvinylpyrrolidon und/oder Vinylpyrrolidon/Vinylacetat-Copolymer | 1,0 bis 10 | 2,0 bis 9,0 | 2,5 bis 8,5 | 2,0 bis 8,0 | 3,0 bis 7,0 |
| Wasser | 80 bis 99 | 82 bis 99 | 84 bis 98 | 87 bis 98 | 90 bis 97 |
| Optionale Additive | add 100 | add 100 | add 100 | add 100 | add 100 |

| | | | | | |
|---|---|---|---|---|---|
| ¹⁾ hydrophob modifiziertes (Meth)acrylsäure Copolymer ²⁾ von dem Copolymer a) verschiedenen Copolymers, welches durch Umsetzung mindestens einer ethylenisch ungesättigten Monomereinheit (b1) mit mindestens einer saccharidischen Monomereinheit (b2) erhalten wird ³⁾ Copolymer a), welches durch Umsetzung - mindestens eines Monomers (a1) aus der Gruppe Acrylsäure, Methacrylsäure, C₁-C₆-Alkylacrylsäureester, C₁-C₆-Alkylmethacrylsäureester, mit - mindestens einem Monomer (a2) aus der Gruppe der der C₁₀₋₃₀Alkyl Acrylate, C₁₀₋₃₀Alkyl Methacrylate, C₁₀-₃₀Alkyl PEG-Acrylate, C₁₀-₃₀Alkyl PEG-Methacrylate oder C₁₀₋₃₀Alkyl PEG-Itaconate erhaltend wird ⁴⁾ Copoymer b), welches durch Umsetzung von N-Vinylpyrrolidon (B1) mit mindestens einem Monomer (B2) aus der Gruppe der Maltodextrine erhalten wird | | | | | |

Wie eingangs ausgeführt zeichnen sich die zuvor beschriebenen kosmetischen Mittel durch besondere haarkosmetische Eigenschaften, insbesondere vorteilhafte Eigenschaften bei der temporären Haarverformung aus. Ein zweiter Gegenstand der vorliegenden Anmeldung ist daher die Verwendung eines erfindungsgemäßen Mittels zur temporären Verformung keratinhaltiger Fasern, insbesondere menschlicher Haare.

Ein dritter Gegenstand der vorliegenden Anmeldung ist ein Verfahren zur temporären Verformung keratinhaltiger Fasern, insbesondere menschlicher Haare, bei welchem die keratinischen Fasern mit einem erfindungsgemäßen kosmetischen Mittel beaufschlagt und temporär in ihrer Form fixiert werden.

## Patentansprüche

1. Kosmetisches Mittel zur temporären Verformung keratinischer Fasern, enthaltend, bezogen auf sein Gesamtgewicht
a) 0,5 bis 3,0 Gew.-% mindestens eines hydrophob modifizierten (Meth)acrylsäure Copolymers, welches durch Umsetzung
- mindestens eines Monomers (a1) aus der Gruppe Acrylsäure. Methacrylsäure, C₁-C₆-Alkylacrylsäureester, C₁-C₆-Alkylmethacrylsäureester, mit
- mindestens einem Monomer (a2) aus der Gruppe der der C₁₀₋₃₀ Alkyl Acrylate, C₁₀₋₃₀ Alkyl Methacrylate erhalten wird;
b) 0,5 bis 5,0 Gew.-% mindestens eines, von dem Copolymer a) verschiedenen Copolymers, welches durch Umsetzung
- mindestens eines Monomers (b1) aus der Gruppe N-Vinylpyrrolidon und N-Vinylformamid, vorzugsweise N-Vinylpyrrolidon, mit
- mindestens einem Monomer (b2) aus der Gruppe der modifizierten Stärken, vorzugsweise der hydrolysierten Stärken, besonders bevorzugt aus der Gruppe der Maltodextrine erhalten wird.

2. Mittel nach Anspruch 1, **dadurch gekennzeichnet, dass** der Gewichtsanteil des Copolymers a) am Gesamtgewicht des kosmetischen Mittels 0,7 bis 2,0 Gew.-%, vorzugsweise 0,9 bis 1,2 Gew.-% beträgt.

3. Mittel nach einem der vorherigen Ansprüche, **dadurch gekennzeichnet, dass** das Copolymer a) aus der Gruppe der Verbindungen mit der INCI Bezeichnung Acrylates/C₁₀₋₃₀ Alkyl Methacrylate Copolymer ausgewählt ist.

4. Mittel nach einem der vorherigen Ansprüche, **dadurch gekennzeichnet, dass** der Gewichtsanteil des Copolymers b) am Gesamtgewicht des kosmetischen Mittels 1,0 bis 4,0 Gew.-%, vorzugsweise 1,5 bis 3,0 Gew.-% beträgt.

5. Mittel nach einem der vorherigen Ansprüche, **dadurch gekennzeichnet, dass** das Copolymer b) aus der Gruppe der Verbindungen mit der INCI Bezeichnung Maltodextrin/VP Copolymer ausgewählt ist.

6. Mittel nach einem der vorherigen Ansprüche, **dadurch gekennzeichnet, dass** es bezogen auf sein Gesamtgewicht weiterhin
c) 1,0 bis 10 Gew.-% Polyvinylpyrrolidon und/oder Vinylpyrrolidon/Vinylacetat-Copolymer, vorzugsweise Polyvinylpyrrolidon enthält.

7. Mittel nach Anspruch 6, **dadurch gekennzeichnet, dass** der Gewichtsanteil des Polyvinylpyrrolidons und/oder Vinylpyrrolidon/Vinylacetat-Copolymers c) am Gesamtgewicht des kosmetischen Mittels 2,0 bis 8,5 Gew.-%, vorzugsweise 3,0 bis 7,0 Gew.-% beträgt.

8. Mittel nach einem der vorherigen Ansprüche, enthaltend, bezogen auf sein Gesamtgewicht
a) 0,9 bis 1,2 Gew.-% eines Copolymer a) aus der Gruppe der Verbindungen mit der INCI Bezeichnung Acrylates/C₁₀₋₃₀ Alkyl Methacrylate Copolymer;
b) 1,5 bis 3,0 Gew.-% eines Copolymers b) aus der Gruppe der Verbindungen mit der INCI Bezeichnung Maltodextrin/VP Copolymer;
c) 3,0 bis 7,0 Gew.-% Polyvinylpyrrolidon.

9. Mittel nach einem der vorherigen Ansprüche, **dadurch gekennzeichnet, dass** das Mittel, bezogen aus sein Gesamtgewicht 80 bis 99 Gew.-%, vorzugsweise 84 bis 98 Gew.-% und insbesondere 90 bis 97 Gew.-% Wasser enthält.

10. Verwendung eines Mittels nach einem der Ansprüche 1 bis 9 zur temporären Verformung keratinhaltiger Fasern, insbesondere menschlicher Haare.

11. Verfahren zur temporären Verformung keratinhaltiger Fasern, insbesondere menschlicher Haare, bei welchem die keratinischen Fasern mit einem kosmetischen Mittel nach einem der Ansprüche 1 bis 9 beaufschlagt und temporär in ihrer Form fixiert werden.

## Claims

1. A cosmetic agent for temporarily shaping keratin fibers, containing, based on the total weight thereof,
a) 0.5 to 3.0 wt.% of at least one hydrophobically modified (meth)acrylic acid copolymer which is obtained by reacting
- at least one monomer (a1) from the group acrylic acid, methacrylic acid, C₁-C₆ alkyl acrylic acid ester, C₁-C₆ alkyl methacrylic acid ester, with
- at least one monomer (a2) from the group of C₁₀₋₃₀ alkyl acrylates, C₁₀₋₃₀ alkyl methacrylates;
b) 0.5 to 5.0 wt.% of at least one copolymer which is different from the copolymer a) and which is obtained by reacting
- at least one monomer (b1) from the group N-vinylpyrrolidone and N-vinylformamide, preferably N-vinylpyrrolidone, with
- at least one monomer (b2) from the group of modified starches, preferably of hydrolyzed starches, particularly preferably from the group of maltodextrins.

2. The agent according to claim 1, **characterized in that** the proportion by weight of the copolymer a) with respect to the total weight of the cosmetic agent is 0.7 to 2.0 wt.%, preferably 0.9 to 1.2 wt.%.

3. The agent according to one of the preceding claims, **characterized in that** the copolymer
a) is selected from the group of compounds having the INCI name acrylates/C₁₀₋₃₀ alkyl methacrylate copolymer.

4. The agent according to one of the preceding claims, **characterized in that** the proportion by weight of the copolymer b) with respect to the total weight of the cosmetic agent is 1.0 to 4.0 wt.%, preferably 1.5 to 3.0 wt.%.

5. The agent according to one of the preceding claims, **characterized in that** the copolymer b) is selected from the group of compounds having the INCI name maltodextrin/VP copolymer.

6. The agent according to one of the preceding claims, **characterized in that** it contains, based on the total weight thereof,
c) 1.0 to 10 wt.% polyvinylpyrrolidone and/or vinylpyrrolidone-vinyl acetate copolymer, preferably polyvinylpyrrolidone.

7. The agent according to claim 6, **characterized in that** the proportion by weight of the polyvinylpyrrolidone and/or vinylpyrrolidone-vinyl acetate copolymer c) with respect to the total weight of the cosmetic agent is 2.0 to 8.5 wt.%, preferably 3.0 to 7.0 wt.%.

8. The agent according to one of the preceding claims, containing, based on the total weight thereof,
a) 0.9 to 1.2 wt.% of a copolymer a) from the group of compounds having the INCI name acrylates/C₁₀₋₃₀ alkyl methacrylate copolymer;
b) 1.5 to 3.0 wt.% of a copolymer b) from the group of compounds having the INCI name maltodextrin/VP copolymer;
c) 3.0 to 7.0 wt.% polyvinylpyrrolidone.

9. The agent according to one of the preceding claims, **characterized in that** the agent contains, based on the total weight thereof, 80 to 99 wt.%, preferably 84 to 98 wt.%, and in particular 90 to 97 wt.%, water.

10. The use of an agent according to one of claims 1 to 9 for temporarily shaping keratin-containing fibers, in particular human hair.

11. A method for temporarily shaping keratin-containing fibers, in particular human hair, wherein the keratin fibers are acted upon by a cosmetic agent according to one of claims 1 to 9 and are temporarily fixed in their shape.

## Revendications

1. Agent cosmétique pour la mise en forme temporaire de fibres kératiniques, contenant, par rapport à son poids total,
a) 0,5 à 3,0 % en poids d'au moins un copolymère d'acide (méth)acrylique hydrophobiquement modifié, qui est obtenu par réaction
- d'au moins un monomère (a1) du groupe acide acrylique, acide méthacrylique, ester d'acide méthacrylique alkyle C₁-C₆, ester d'acide méthacrylique alkyle C₁-C6, ayant
- au moins un monomère (a2) du groupe constitué par l'acrylate alkyle C₁₀₋₃₀, le méthacrylate alkyle C₁₀₋₃₀ ;
b) 0,5 à 5,0 % en poids d'au moins un copolymère autre que le copolymère a), qui est obtenu par réaction
- d'au moins un monomère (b1) du groupe comprenant la N-vinylpyrrolidone et le N-vinylformamide, de préférence la N-vinylpyrrolidone, ayant
- au moins un monomère (b2) du groupe des amidons modifiés, de préférence des amidons hydrolysés, en particulier de préférence du groupe des maltodextrines.

2. Agent selon la revendication 1, **caractérisé en ce que** la proportion en poids du copolymère a) par rapport au poids total de l'agent cosmétique est de 0,7 à 2,0 % en poids, de préférence 0,9 à 1,2%.

3. Agent selon l'une des revendications précédentes, **caractérisé en ce que** le copolymère
a) est choisi dans le groupe des composés ayant la nomenclature INCI Acrylates/C₁₀₋₃₀ Copolymère de méthacrylate d'alkyle.

4. Agent selon l'une des revendications précédentes, **caractérisé en ce que** la proportion en poids du copolymère b) par rapport au poids total de l'agent cosmétique est de 1,0 à 4,0 % en poids, de préférence 1,5 à 3,0 % en poids.

5. Agent selon l'une des revendications précédentes, **caractérisé en ce que** le copolymère b) est choisi dans le groupe des composés ayant la nomenclature INCI copolymère maltodextrine/VP.

6. Agent selon l'une des revendications précédentes, **caractérisé en ce qu'**il contient, par rapport à son poids total, en outre
c) 1,0 à 10 % en poids de polyvinylpyrrolidone et/ou de copolymère vinylpyrrolidone-acétate de vinyle, de préférence de polyvinylpyrrolidone.

7. Agent selon la revendication 6, **caractérisé en ce que** la proportion en poids du copolymère polyvinylpyrrolidone et/ou vinylpyrrolidone/acétate de vinyle c) par rapport au poids total de l'agent cosmétique est de 2,0 à 8,5 % en poids, de préférence 3,0 à 7,0 % en poids.

8. Agent selon l'une des revendications précédentes contenant, par rapport à son poids total
a) 0,9 à 1,2 % en poids d'un copolymère a) du groupe des composés ayant la nomenclature INCI Acrylates/C10-30 Copolymère de méthacrylate d'alkyle ;
b) 1,5 à 3,0 % en poids d'un copolymère b) du groupe des composés ayant la nomenclature INCI Copolymère maltodextrine/VP ;
c) 3,0 à 7,0 % en poids de polyvinylpyrrolidone.

9. Agent selon l'une des revendications précédentes, **caractérisé en ce que** ledit agent contient, par rapport à son poids total, 80 à 99 % en poids, de préférence 84 à 98 % en poids et en particulier 90 à 97 % en poids d'eau.

10. Utilisation d'un agent selon l'une des revendications 1 à 9 pour la mise en forme temporaire de fibres kératiniques, en particulier de cheveux humains.

11. Procédé de déformation temporaire de fibres kératiniques, en particulier de cheveux humains, selon lequel les fibres kératiniques sont sollicitées par un agent cosmétique selon l'une des revendications 1 à 9 et sont fixées temporairement dans leur forme.
